# FASCICULE DE BREVET EUROPEEN

(11) **EP 0 977 552 B1**
(45) Date de publication et mention de la délivrance du brevet: **11.08.2004**
(21) Numéro de dépôt: 98951551.5
(22) Date de dépôt: 26.10.1998
(51) Int. Cl.: A61K 7/48, C07C 257/12

(54) **COMPOSITION POUR APPLICATION TOPIQUE SUR LA PEAU ET/OU SES PHANERES COMPRENANT AU MOINS UN COMPOSE COMPORTANT UN FRAGMENT IMINOPHENOL**
ZUSAMMENSETZUNG ZUR TOPISCHEN ANWENDUNG AUF DIE HAUT UND DEREN KOERPERANHAENGSELN; DIE MINDESTENS EINE VERBINDUNG AUF IMINOPHENOLBASIS ENTHAELT
COMPOSITION FOR TOPICAL APPLICATION ON THE SKIN AND/OR ITS APPENDAGES COMPRISING AT LEAST A COMPOUND CONTAINING AN IMINOPHENOL FRAGMENT

(30) Priorité: 04.11.1997 FR 9713843
(43) Date de publication de la demande: 09.02.2000
(73) Titulaire: L'OREAL, 75008 Paris (FR)
(72) Inventeur: TULOUP, Rémy, F-75014 Paris (FR); BLAISE, Christian, F-94160 Saint Mande (FR)
(74) Mandataire: Renard, Emmanuelle
(86) Numéro de dépôt international: PCT/FR1998/002284
(87) Numéro de publication internationale: WO 1999/022707

(56) Documents cités:
- US-A- 2 220 065
- US-A- 3 182 053
- WOODRUFF J: "LIGHTENING SKIN AND LESSENING CELLULITE" MANUFACTURING CHEMIST, vol. 67, no. 4, avril 1996, page 38/39, 41 XP000683051
- W.W. KAEDING ET AL.: "Schiff base phenyl N-methylcarbamates" JOURNAL OF AGRICULTURAL AND FOOD CHEMISTRY., vol. 13, no. 4, 1965, pages 378-380, XP002069594 WASHINGTON US

## Description

La présente invention se rapporte à une composition pour application topique sur la peau et/ou ses phanères comprenant, dans un milieu cosmétiquement et/ou dermatologiquement acceptable, au moins un composé comportant un fragment iminophénol et à l'utilisation dudit composé comme agent dépigmentant ou blanchissant dans une composition cosmétique et/ou dermatologique.

La couleur de la peau humaine est fonction de différents facteurs et notamment des saisons de l'année, de la race et du sexe, et elle est principalement déterminée par la nature et la concentration de mélanine produite par les mélanocytes. Les mélanocytes sont les cellules spécialisées qui par l'intermédiaire d'organelles particuliers, les mélanosomes, synthétisent la mélanine. En outre, à différentes périodes de leur vie, certaines personnes voient apparaître sur la peau et plus spécialement sur les mains, des taches plus foncées et/ou plus colorées, conférant à la peau une hétérogénéité. Ces taches sont dues aussi à une concentration importante de mélanine dans les kératinocytes situés à la surface de la peau.

De la même manière, la couleur des poils et des cheveux est due à la mélanine, lorsque les poils ou les cheveux sont foncés, certaines personnes désirent voir ceux-ci plus clairs. Ceci est particulièrement intéressant pour les poils qui sont moins visibles lorsqu'ils sont clairs que lorsqu'ils sont foncés.

Le mécanisme de formation de la pigmentation de la peau, des poils et des cheveux, c'est-à-dire de la formation de la mélanine est particulièrement complexe et fait intervenir schématiquement les principales étapes suivantes :
Tyrosine → Dopa → Dopaquinone → Dopachrome → Mélanine
La tyrosinase (monophénol dihydroxyl phénylalanine : oxygen oxydo-reductase EC 1.14.18.1) est l'enzyme essentielle intervenant dans cette suite de réactions. Elle catalyse notamment la réaction de transformation de la tyrosine en Dopa (dihydroxyphénylalanine) grâce à son activité hydroxylase et la réaction de transformation de la Dopa en dopaquinone grâce à son activité oxydase. Cette tyrosinase n'agit que lorsqu'elle est à l'état de maturation sous l'action de certains facteurs biologiques.

Une substance est reconnue comme dépigmentante si elle agit directement sur la vitalité des mélanocytes épidermiques où se déroule la mélanogénèse et/ou si elle interfère avec une des étapes de la biosynthèse de la mélanine soit en inhibant une des enzymes impliquées dans la mélanogénèse soit en s'intercalant comme analogue structural d'un des composés chimiques de la chaîne de synthèse de la mélanine, chaîne qui peut alors être bloquée et ainsi assurer la dépigmentation.

Les substances les plus utilisées en tant que dépigmentants sont plus particulièrement l'hydroquinone et ses dérivés, en particulier ses éthers tels que le monométhyléther et le monoéthyléther d'hydroquinone. Ces composés, bien qu'ils présentent une efficacité certaine, ne sont malheureusement pas exempts d'effets secondaires du fait de leur toxicité, ce qui peut rendre leur emploi délicat, voire dangereux. Cette toxicité provient de ce qu'ils interviennent sur des mécanismes fondamentaux de la mélanogénèse en tuant des cellules qui risquent alors de perturber leur environnement biologique et qui par conséquent obligent la peau à les évacuer en produisant des toxines.

Ainsi, l'hydroquinone est un composé particulièrement irritant et cytotoxique pour le mélanocyte, dont le remplacement, total ou partiel a été envisagé par de nombreux auteurs.

On a ainsi cherché des substances qui n'interviennent pas dans le mécanisme de la mélanogénèse mais qui agissent en amont sur la tyrosinase en empêchant son activation et sont de ce fait beaucoup moins toxiques. On utilise couramment comme inhibiteur de l'activation de la tyrosinase l'acide kojique qui complexe le cuivre présent dans le site actif de cette enzyme. Malheureusement, ce composé peut provoquer des réactions d'allergie ("Contact allergy to kojic acid in skin care products", Nakagawa M. et al., in Contact Dermatitis, Jan. 95, Vol 42 (1), pp.9-13). Ce composé est également instable en solution, ce qui complique quelque peu la fabrication de la composition.

L'utilisation de substances dépigmentantes topiques inoffensives présentant une bonne efficacité est tout particulièrement recherchée en vue de traiter les hyperpigmentations régionales par hyperactivité mélanocytaire telles que les mélasmas idiopathiques, survenant lors de la grossesse ("masque de grossesse" ou chloasma) ou d'une contraception oestro-progestative, les hyperpigmentations localisées par hyperactivité et prolifération mélanocytaire bénigne, telles que les taches pigmentaires séniles dites lentigo actiniques, les hyperpigmentations ou dépigmentations accidentelles, éventuellement dues à la photosensibilisation ou à la cicatrisation post-lésionnelle, ainsi que certaines leucodermies, telles que le vitiligo. Pour ces dernières (les cicatrisations pouvant aboutir à une cicatrice donnant à la peau un aspect plus blanc et les leucodermies), à défaut de pouvoir repigmenter la peau lésée, on achève de dépigmenter les zones de peau normale résiduelle pour donner à l'ensemble de la peau une teinte blanche homogène.

Aussi, il subsiste le besoin d'un nouvel agent blanchissant de la peau humaine, des poils et/ou des cheveux à action aussi efficace que ceux connus, mais n'ayant pas leurs inconvénients, c'est-à-dire qui soit non irritant, non toxique et/ou non allergisant pour la peau et stable dans une composition.

La demanderesse a trouvé de manière inattendue que des composés comportant un fragment iminophénol présentent une activité dépigmentante, même à faibles concentrations, sans faire preuve de cytotoxicité.

On connaît de US-A-3 182 053 des composés à fragment iminophénol utilisables comme anti-pathogènes, anti-viraux ou anti-bactériens, qui sont destinés à être administrés par voie orale ou parentérale. On connaît en outre de US-A-2 220 065 certains composés à fragment iminophénol utilisables comme anti-oxydants pour l'essence, le caoutchouc et d'autres matériaux auto-oxydables.

Toutefois, à la connaissance de la Demanderesse, il n'a encore jamais été proposé d'administrer des composés à fragment iminophénol par voie topique, en particulier en vue de blanchir ou dépigmenter la peau ou ses phanères. Seuls des dérivés du paracétamol (WO-98/38978), ou des dérivés d'oxamate (EP-0 898 956) ont été utilisés dans cette optique.

La présente invention a donc pour objet une composition pour application topique sur la peau et/ou ses phanères comprenant, dans un milieu cosmétiquement et/ou dermatologiquement acceptable, au moins un composé de formule (I) suivante : 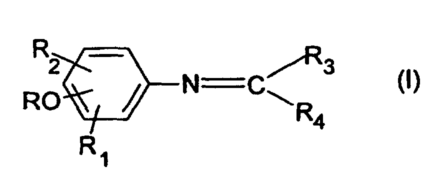 dans laquelle :
- R représente un groupement choisi parmi :
   - l'atome d'hydrogène ;
   - un groupement alkyle en C₁-C₂₄ linéaire ramifié ou cyclique, saturé ou insaturé, éventuellement hydroxylé par une ou plusieurs fonctions hydroxyles ;
   - un groupement aryle, substitué ou non par une ou plusieurs fonctions choisies parmi : -OH ; NH₂ ; -COOH ; -NO₂ ; -OR₅ avec R₅ = alkyle en C₁-C₂₄ ; -COOR₆ avec R₆ = alkyle en C₁-C₂₄ ; -NR₇R₈ avec R₇ = H ou alkyle en C₁-C₂₄, R₈ = H ou alkyle en C₁-C₂₄ ;
   - un groupement -COR₉, R₉ représentant un groupement alkyle en C₁-C₂₄ linéaire, ramifié ou cyclique, saturé ou insaturé, éventuellement hydroxylé par une ou plusieurs fonctions hydroxyles, un groupement aryle substitué ou non par une ou plusieurs fonctions choisies parmi -OH, -NH₂, -COOH, -NO₂, -OR₅, -COOR₆, -NR₇R₈dans lesquelles R₅, R₆, R₇ et R₈ ont la même définition que ci-dessus.
- R₁, R₂, identiques ou différents représentent un groupement choisi parmi :
   - l'atome d'hydrogène ;
   - un groupement alkyle en C₁-C₂₄, linéaire, ramifié ou cyclique, saturé ou insaturé, éventuellement hydroxylé par une ou plusieurs fonctions hydroxyles ;
   - un groupement aryle, substitué ou non par une ou plusieurs fonctions choisies parmi -OH, -NH₂, -COOH, -NO₂, -OR₅, -COOR₆, -NR₇R₈ dans lesquelles R₅, R₆, R₇ et R₈ ont la même définition que ci-dessus;
   - un groupement choisi parmi : -OH ; -OQ₁ ; -COQ₂ ; -COOQ₃ ; -NQ₄Q₅ ; -CONQ₆Q₇ ; -SQ₈ ; -CH₂OQ₉ ; Q₁, Q₂, Q₃, Q₄, Q₅, Q₆, Q₇, Q₈ et Q₉ étant choisis parmi l'atome d'hydrogène, les groupements alkyles en C₁₋C₂₄, linéaires, ramifiés ou cycliques, saturés ou insaturés, éventuellement substitués par un ou plusieurs groupements hydroxyles, les aryles substitués ou non par une ou plusieurs fonctions choisies parmi : -OH, -NH₂, -COOH, -NO₂, -OR₅, -COOR₆, -NR₇R₈ dans lesquelles R₅, R₆, R₇ et R₈ ont la même définition que ci-dessus ;
   - les résidus d'acides aminés et de carbohydrates cycliques ou non cycliques,
- R₃ représente un atome d'hydrogène,
- R₄ représente un groupement choisi parmi :
   - l'atome d'hydrogène
   - un groupement aryle, substitué ou non par une ou plusieurs fonctions choisies parmi -OH, -NH₂, -COOH, -NO₂, -OR₅, -COOR₆, -NR₇R₈ dans lesquelles R₅, R₆, R₇ et R₈ ont la même définition que ci-dessus;
   - un groupement choisi parmi : -OH ; -OQ₁ ; -COQ₂; -COOQ₃ ; -NQ₄Q₅ ; -CONQ₆Q₇ ; -SQ₈ ; -CH₂OQ₉ ; Q₁, Q₂, Q₃, Q₄, Q₅, Q₆, Q₇, Q₈ et Q₉ étant choisis parmi l'atome d'hydrogène, les groupements alkyles en C₁-C₂₄, linéaires, ramifiés ou cycliques, saturés ou insaturés, éventuellement substitués par un ou plusieurs groupements hydroxyles, les aryles substitués ou non par une ou plusieurs fonctions choisies parmi : -OH, -NH₂, -COOH, -NO₂, -OR₅, -COOR₆, -NR₇R₈ dans lesquelles R₅, R₆, R₇ et R₈ ont la même définition que ci-dessus ;
   - les résidus d'acides aminés et de carbohydrates cycliques ou non cycliques.

Ces composés présentent l'avantage d'être faciles à obtenir. Ils peuvent être notamment obtenus en faisant réagir un aminophénol avec un composé carbonylé : cétone ou aldéhyde. Le réactif carbonylé peut éventuellement être utilisé sous forme d'acétal.

Selon la présente invention, parmi les radicaux alkyles linéaires ou ramifiés ayant de 1 à 24 atomes de carbone, on peut citer avantageusement les radicaux méthyle, éthyle, propyle, isopropyle, butyle, tertiobutyle, hexyle, octyle, nonyle, 2-éthyl-hexyle et dodécyle.

Parmi les radicaux alkyles linéaires ayant de 1 à 24 atomes de carbone, on peut citer notamment les radicaux méthyle, éthyle, propyle, octyle, dodécyle, hexadécyle, béhényle et octadécyle.

Parmi les radicaux alkyles ramifiés ayant de 1 à 24 atomes de carbone, on peut citer notamment les radicaux 2-éthyl-hexyle, 2-butyl-octyle, 2-hexyl-décyle.

Parmi les radicaux alkyles insaturés, on peut citer plus particulièrement le radical allyle.

Lorsque le radical alkyle est cyclique, on peut notamment citer le radical cyclohexyle, cholestéryle ou terbutylcyclohexyle.

De préférence, les composés de formule (I) de la présente invention sont ceux pour lesquels l'une au moins et de préférence toutes les conditions ci-dessous sont respectées :
- R représente un atome d'hydrogène,
- OR est en position ortho ou para par rapport à la fonction imine,
- R₃ = H,
- R₄ représente un groupement -NQ₄Q₅ tel que défini ci-dessus.

Le composé de formule (I) préféré est le N'-(4-hydroxyphényl)-N,N-diméthyl formamidine.

Par rapport aux composés de l'art antérieur connus comme dépigmentants, les composés de formule (I) présentent l'avantage d'être plus stables et plus efficaces, comme il sera montré dans les tests ci-dessous.

La composition cosmétique ou dermatologique selon l'invention est avantageusement destinée à dépigmenter et/ou blanchir la peau humaine et/ou enlever les taches pigmentaires de la peau et/ou dépigmenter les poils et/ou les cheveux.

La présente invention a aussi pour objet l'utilisation des composés de formule (I) précités dans une composition cosmétique, comme inhibiteur de la tyrosinase et/ou de la synthèse de la mélanine.

Elle a également pour objet l'utilisation de ces composés de formule (I) pour la fabrication d'une composition dermatologique destinée à dépigmenter et/ou blanchir la peau humaine et/ou enlever les taches pigmentaires de la peau et/ou dépigmenter les poils et/ou les cheveux.

La présente invention a aussi pour objet l'utilisation de ces composés de formule (1) dans une composition cosmétique dépigmentante et/ou blanchissante de la peau humaine, des poils ou des cheveux.

La présente invention se rapporte également à un procédé cosmétique de dépigmentation et/ou de blanchiment de la peau humaine, des poils ou des cheveux consistant à appliquer sur la peau, les poils ou les cheveux une composition cosmétique selon l'invention.

La composition selon l'invention est appropriée pour une utilisation topique et contient donc un milieu cosmétiquement ou dermatologiquement acceptable, c'est-à-dire compatible avec la peau, les poils ou les cheveux.

Les dérivés de formule (1) peuvent être notamment présents dans la composition en une quantité allant de 0,001 à 10% et de préférence de 0,005 à 5% du poids total de la composition.

La composition de l'invention peut se présenter sous toutes les formes galéniques normalement utilisées pour une application topique, notamment sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide, d'une dispersion d'huile dans une phase aqueuse à l'aide de sphérules, ces sphérules pouvant être des nanoparticules polymériques telles que les nanosphères et les nanocapsules ou mieux des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau ou sur les cheveux sous forme d'aérosol. Elle peut également se présenter sous forme solide, et par exemple sous forme de stick. Elle peut être utilisée comme produit de soin et/ou comme produit de maquillage. Elle peut également être sous une forme de shampooings ou après-shampooings.

De façon connue, la composition de l'invention peut contenir également les adjuvants habituels dans les domaines cosmétique et dermatologique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans les domaines considérés, et par exemple de 0,01 à 20 % du poids total de la composition. Ces adjuvants, selon leur nature, peuvent être introduits dans la phase grasse, dans la phase aqueuse, dans les vésicules lipidiques et/ou dans les nanoparticules.

Lorsque la composition de l'invention est une émulsion, la proportion de la phase grasse peut aller de 5 à 80% en poids, et de préférence de 5 à 50% en poids par rapport au poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 à 30 % en poids, et de préférence de 0,5 à 20 % en poids par rapport au poids total de la composition.
Comme huiles utilisables dans l'invention, on peut citer les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras (alcool cétylique), des acides gras, des cires (cire de carnauba, ozokérite).

Comme émulsionnants et coémulsionnants utilisables dans l'invention, on peut citer par exemple les esters d'acide gras et de polyéthylène glycol tels que le stéarate de PEG-20, et les esters d'acide gras et de glycérine tels que le stéarate de glycéryle.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Comme actifs, on peut utiliser notamment les polyols (glycérine, propylène glycol), les vitamines, les agents kératolytiques et/ou desquamants (acide salicylique et ses dérivés, alpha-hydroxyacides, acide ascorbique et ses dérivés), les agents anti-inflammatoires, les agents apaisants et leurs mélanges. On peut également associer les composés de formule (I) à d'autres agents dépigmentants, tels que l'acide kojique ou l'hydroquinone et ses dérivés, ce qui permet d'utiliser ces derniers à des doses moins toxiques pour la peau. En cas d'incompatibilité, ces actifs et/ou les composés de formule (I) peuvent être incorporés dans des sphérules, notamment des vésicules ioniques ou non-ioniques et/ou des nanoparticules (nanocapsules et/ou nanosphères), de manière à les isoler les uns des autres dans la composition.

L'invention va maintenant être illustrée à l'aide des exemples qui suivent. Les concentrations sont données en pourcentage en poids.

### Exemple de composé

### Préparation du N'-(4-hydroxyphényl)-N,N-diméthyl formamidine :

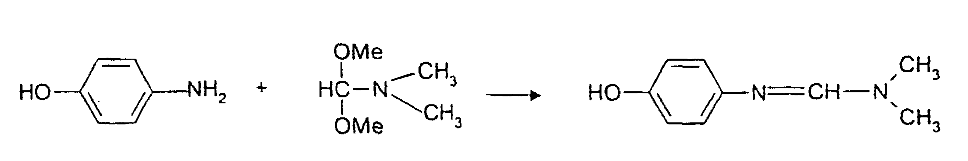

On introduit 10 g de para aminophénol et 13,4 ml de N, N-diméthyl formamide diméthyl acétal dans 100 ml de méthanol et on porte le mélange au reflux pendant 30 minutes. Après refroidissement, le N'-(4-hydroxyphényl)-N,N-diméthyl formamidine précipite. Il est recristallisé dans l'éthanol. La température de fusion du produit obtenu est de 218°C. L'analyse élémentaire est conforme à la structure attendue.

### Tests :

Un test biologique a mis en évidence l'activité dépigmentante des composés de formule (I)

Ce test correspond à celui décrit dans le brevet FR 2734825 déposé par la Demanderesse, ainsi que dans l'article de R. Schmidt, P. Krien et M. Régnier, Anal. Biochem., 235(2), 113-18, (1996). Ce test est ainsi réalisé sur coculture de kératinocytes et de mélanocytes.

Pour chaque composé testé, il est déterminé la valeur de IC50 qui correspond à la concentration micromolaire (µM) pour laquelle est observée 50% d'inhibition de la mélanogénèse.

Par ailleurs, une classe est donnée à chacun de ces composés pour leur activité dépigmentante maximale :
classe 1 : 10 à 30% d'inhibition de la mélanogénèse par rapport au témoin (même expérience sans composé à tester) ;
classe 2 : 30 à 60% d'inhibition de la mélanogénèse par rapport au témoin (même expérience sans composé à tester) ;
classe 3 : 60 à 100% d'inhibition de la mélanogénèse par rapport au témoin (même expérience sans composé à tester).

Les résultats sont rassemblés dans le tableau suivant :

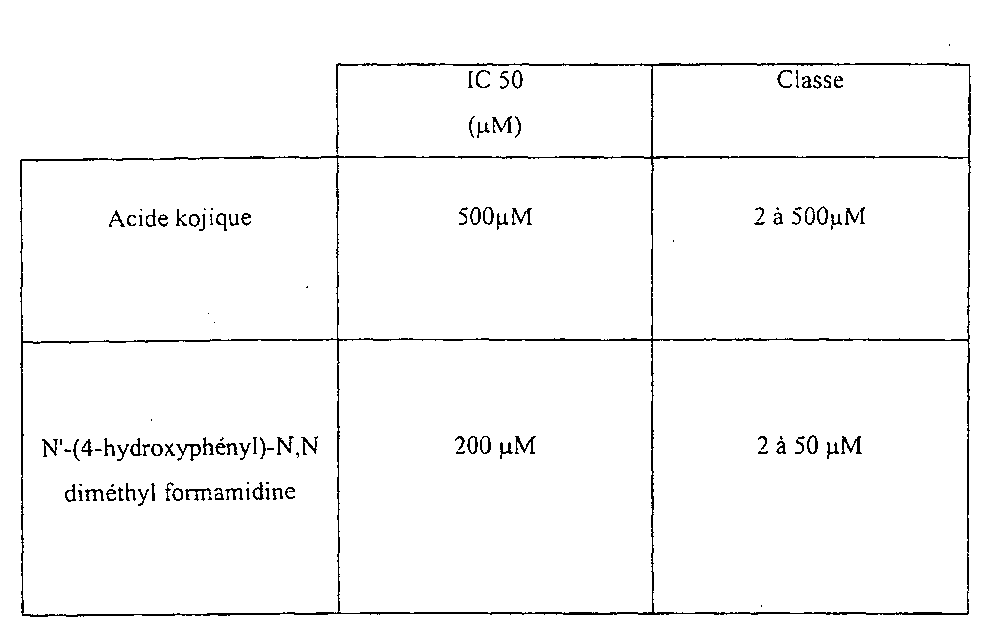

Ces composés de formule (I) présentent donc une plus grande efficacité dépigmentante que l'acide kojique. En outre, ils ont l'avantage de ne pas présenter de cytotoxicité à l'égard des kératinocytes et des mélanocytes, défaut majeur des dépigmentants déjà connus.

### Exemples de compositions

### Exemple 1 : Crème traitante

- Alcool cétylique 1,05 %
- Stéarate de PEG-20 (Myrj 49 vendu par la société ICI) 2 %
- Cyclométhicone 6 %
- N'-(4-hydroxyphényl)-N,N diméthyl formamidine 0,5 %
- Carbomer 0,6 %
- Glycérine 3 %
- Triéthanolamine 1 %
- Conservateurs 0,5 %
- Eau déminéralisée qsp 100 %

La crème obtenue utilisée en application quotidienne, permet d'obtenir un blanchiment de la peau.

### Exemple 2 : Gel traitant

- Propylène glycol 10 %
- Alcool éthylique 40 % Glycérine 3 %
- N'-(4-hydroxyphényl)-N,N diméthyl formamidine 0,5 %
- Conservateurs 0,15 %
- Parfum 0,15 %
- Eau déminéralisée qsp 100 %

Le gel obtenu peut être utilisé quotidiennement et est apte à dépigmenter la peau.

### Exemple 3 : Stick traitant

- Cire de Carnauba 5 %
- Ozokerite 7 %
- Lanoline 6 %
- Dioxyde de titane (pigments) 20 %
- Amidon de riz (charge) 7 %
- EDTA 0,1 %
- N'-(4-hydroxyphényl)-N,N diméthyl formamidine 2%
- Perhydrosqualène qsp 100%

Le stick obtenu, utilisé sur les taches pigmentaires, permet de les atténuer voire de les faire disparaître.

## Revendications

1. Composition adaptée à une application topique sur la peau et/ou ses phanères comprenant, dans un milieu cosmétiquement et/ou dermatologiquement acceptable, au moins un dérivé de formule (I) suivante : 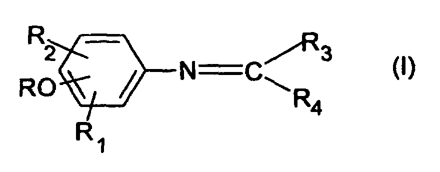 dans laquelle :
• R représente un groupement choisi parmi :
- l'atome d'hydrogène ;
- un groupement alkyle en C₁-C₂₄ linéaire ramifié ou cyclique, saturé ou insaturé, éventuellement hydroxylé par une ou plusieurs fonctions hydroxyles ;
- un groupement aryle, substitué ou non par une ou plusieurs fonctions choisies parmi : -OH ; NH₂ ; -COOH ; -NO₂ ; -OR₅ avec R₅ = alkyle en C₁-C₂₄ ; -COOR₆ avec R₆ = alkyle en C₁-C₂₄ ; -NR₇R₈ avec R₇ = H ou alkyle en C₁-C₂₄, R₈ = H ou alkyle en C₁-C₂₄ ;
- un groupement -COR₉, R₉ représentant un groupement alkyle en C₁-C₂₄ linéaire, ramifié ou cyclique, saturé ou insaturé, éventuellement hydroxylé par une ou plusieurs fonctions hydroxyles, un groupement aryle substitué ou non par une ou plusieurs fonctions choisies parmi -OH, -NH₂, -COOH, -NO₂, -OR₅, -COOR₆, -NR₇R₈ dans lesquelles R₅, R₆, R₇ et R₈ ont la même définition que ci-dessus.
• R₁, R₂, identiques ou différents représentent un groupement choisi parmi :
- l'atome d'hydrogène ;
- un groupement alkyle en C₁-C₂₄, linéaire, ramifié ou cyclique, saturé ou insaturé, éventuellement hydroxylé par une ou plusieurs fonctions hydroxyles ;
- un groupement aryle, substitué ou non par une ou plusieurs fonctions choisies parmi -OH, -NH₂, -COOH, -NO₂, -OR₅, -COOR₆, -NR₇R₈ dans lesquelles R₅, R₆, R₇ et R₈ ont la même définition que ci-dessus;
- un groupement choisi parmi : -OH ; -OQ₁ ; -COQ₂ ; -COOQ₃ ; -NQ₄Q₅ ; - CONQ₆Q₇ ; -SQ₈; -CH₂OQ₉ ; Q₁, Q₂, Q₃, Q₄, Q₅, Q₆, Q₇, Q₈ et Q₉ étant choisis parmi l'atome d'hydrogène, les groupements alkyles en C₁-C₂₄, linéaires, ramifiés ou cycliques, saturés ou insaturés, éventuellement substitués par un ou plusieurs groupements hydroxyles, les aryles substitués ou non par une ou plusieurs fonctions choisies parmi : -OH, -NH₂, -COOH, -NO₂, -OR₅, -COOR₆, -NR₇R₈ dans lesquelles R₅, R₆, R₇ et R₈ ont la même définition que ci-dessus ;
- les résidus d'acides aminés et de carbohydrates cycliques ou non cycliques,
• R₃ représente un atome d'hydrogène,
• R₄ représente un groupement choisi parmi :
- l'atome d'hydrogène,
- un groupement aryle, substitué ou non par une ou plusieurs fonctions choisies parmi -OH, -NH₂, -COOH, -NO₂, -OR₅, -COOR₆, -NR₇R₈ dans lesquelles R₅, R₆, R₇ et R₈ ont la même définition que ci-dessus;
- un groupement choisi parmi : -OH ; -OQ₁ ; -COQ₂ ; -COOQ₃ ; -NQ₄Q₅ ; -CONQ₆Q₇ ; -SQ₈ ; -CH₂OQ₉ ; Q₁, Q₂, Q₃, Q₄, Q₅, Q₆, Q₇, Q₈ et Q₉ étant choisis parmi l'atome d'hydrogène, les groupements alkyles en C₁-C₂₄, linéaires, ramifiés ou cycliques, saturés ou insaturés, éventuellement substitués par un ou plusieurs groupements hydroxyles, les aryles substitués ou non par une ou plusieurs fonctions choisies parmi : -OH, -NH₂, -COOH, -NO₂, -OR₅, -COOR₆, -NR₇R₈ dans lesquelles R₅, R₆, R₇ et R₈ ont la même définition que ci-dessus ;
- les résidus d'acides aminés et de carbohydrates cycliques ou non cycliques.

2. Composition selon la revendication 1, **caractérisée en ce que** ledit dérivé présente l'une au moins et de préférence toutes les conditions ci-dessous :
- R représente un atome d'hydrogène,
- OR est en position ortho ou para par rapport à la fonction imine,
- R₄ représente un groupement -NQ₄Q₅ tel que défini dans la revendication 1.

3. Composition selon la revendication 1 ou 2, **caractérisée en ce que** ledit dérivé est le N'-(4-hydroxyphényl)-N,N-diméthyl formamidine.

4. Composition selon l'une quelconque des revendications 1 à 3, **caractérisée en ce que** le dérivé de formule (I) est présent en une quantité allant de 0,001 à 10% et de préférence de 0,005 à 5 % du poids total de la composition.

5. Composition selon l'une quelconque des revendications 1 à 4, **caractérisée en ce que** la composition comprend en outre, au moins un actif choisi parmi les agents kératolytiques et/ou desquamants, anti-inflammatoires, apaisants, autres agents dépigmentants et leurs mélanges.

6. Utilisation d'au moins un dérivé de formule (I), tel que défini dans l'une des revendications 1 à 3, dans une composition cosmétique dépigmentante et/ou blanchissante de la peau humaine, des poils ou des cheveux.

7. Utilisation d'au moins un dérivé de formule (I), tel que défini dans l'une des revendications 1 à 3, dans une composition cosmétique, comme inhibiteur de la tyrosinase et/ou de la synthèse de la mélanine.

8. Utilisation d'au moins un dérivé de formule (I), tel que défini dans l'une des revendications 1 à 3, pour la fabrication d'une composition dermatologique destinée à dépigmenter et/ou blanchir la peau humaine et/ou enlever les taches pigmentaires de la peau et/ou dépigmenter les poils et/ou les cheveux.

9. Procédé cosmétique de dépigmentation et/ou blanchiment de la peau humaine, des poils ou des cheveux, **caractérisé en ce qu'**il consiste à appliquer sur la peau, les poils ou les cheveux une composition selon l'une des revendications précédentes 1 à 5.

## Patentansprüche

1. Für eine topische Anwendung auf die Haut und/ oder die Hautanhangsgebilde geeignete Zusammensetzung, die in einem kosmetisch und/oder dermatologisch akzeptablen Medium mindestens eine Verbindung der folgenden Formel (I) enthält: 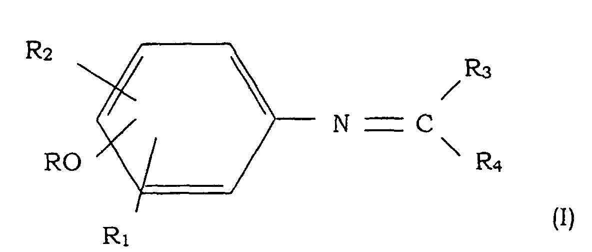 worin bedeuten:
· R eine Gruppe, die ausgewählt ist unter:
- einem Wasserstoffatom;
- einer geradkettigen, verzweigten oder cyclischen, gesättigten oder ungesättigten, gegebenenfalls mit einer oder mehreren Hydroxyfunktionen hydroxylierten C₁₋₂₄-Alkylgruppe;
- einer Arylgruppe, die gegebenenfalls mit einer oder mehreren Funktionen substituiert ist, die unter den folgenden funktionellen Gruppen ausgewählt sind: -OH, -NH₂, -COOH, -NO₂, -OR₅ mit R₅ = C₁₋₂₄-Alkyl, -COOR₆ mit R₆ = C₁₋₂₄-Alkyl, -NR₇R₈ mit R₇ = H oder C₁₋₂₄-Alkyl und R₈ = H oder C₁₋₂₄-Alkyl;
- einer Gruppe -COR₉, wobei R₉ bedeutet: eine geradkettige, verzweigte oder cyclische, gesättigte oder ungesättigte, gegebenenfalls mit einer oder mehreren Hydroxyfunktionen hydroxylierte C₁₋₂₄-Alkylgruppe, eine Arylgruppe, die gegebenenfalls mit einer oder mehreren Funktionen substituiert ist, die unter den folgenden funktionellen Gruppen ausgewählt sind: -OH, -NH₂, -COOH, -NO₂, -OR₅, -COOR₆, -NR₇R₈, wobei R₅, R₆, R₇ und R₈ die oben angegebenen Bedeutungen aufweisen;
· R₁ und R₂, die gleich oder verschieden sind, eine Gruppe, die ausgewählt ist unter:
- einem Wasserstoffatom;
- einer geradkettigen, verzweigten oder cyclischen, gesättigten oder ungesättigten, gegebenenfalls mit einer oder mehreren Hydroxyfunktionen hydroxylierten C₁₋₂₄-Alkylgruppe;
- einer Arylgruppe, die gegebenenfalls mit einer oder mehreren Funktionen substituiert ist, die unter den folgenden funktionellen Gruppen ausgewählt sind: -OH, -NH₂, -COOH, -NO₂, -OR₅, -COOR₆, -NR₇R₈, wobei R₅, R₆, R₇ und R₈ die oben angegebenen Bedeutungen aufweisen;
- eine Gruppe, die ausgewählt ist unter: -OH, -OQ₁, -COQ₂, -COOQ₃, -NQ₄Q₅, -CONQ₆Q₇, -SQ₈ und -CH₂OQ₉, wobei die Gruppen Q₁, Q₂, Q₃, Q₄, Q₅, Q₆, Q₇, Q₈ und Q₉ ausgewählt sind unter Wasserstoff, geradkettigen, verzweigten oder cyclischen, gesättigten oder ungesättigten, gegebenenfalls mit einer oder mehreren Hydroxyfunktionen hydroxylierten C₁₋₂₄-Alkylgruppen, Arylgruppen, die gegebenenfalls mit einer oder mehreren Funktionen substituiert sind, die unter den folgenden funktionellen Gruppen ausgewählt sind: -OH, -NH₂, -COOH, -NO₂, -OR₅, -COOR₆, -NR₇R₈, wobei R₅, R₆, R₇ und R₈ die oben angegebenen Bedeutungen aufweisen;
- Aminosäureresten und cyclischen oder nicht cyclischen Kohlenhydraten;
· R₃ ein Wasserstoffatom;
· R₄ eine Gruppe, die ausgewählt ist unter:
- einem Wasserstoffatom;
- einer Arylgruppe, die gegebenenfalls mit einer oder mehreren Funktionen substituiert ist, die unter den folgenden funktionellen Gruppen ausgewählt sind: -OH, -NH₂, -COOH, - NO₂, -OR₅, -COOR₆, -NR₇R₈, wobei R₅, R₆, R₇ und R₈ die oben angegebenen Bedeutungen aufweisen;
- einer Gruppe, die ausgewählt ist unter: -OH, -OQ₁, -COQ₂, -COOQ₃, -NQ₄Q₅, -CONQ₆Q₇, -SQ₈ und -CH₂OQ₉, wobei die Gruppen Q₁, Q₂, Q₃, Q₄, Q₅, Q₆, Q₇, Q₈ und Q₉ ausgewählt sind unter Wasserstoff, geradkettigen, verzweigten oder cyclischen, gesättigten oder ungesättigten, gegebenenfalls mit einer oder mehreren Hydroxyfunktionen hydroxylierten C₁₋₂₄-Alkylgruppen, Arylgruppen, die gegebenenfalls mit einer oder mehreren Funktionen substituiert sind, die unter den folgenden funktionellen Gruppen ausgewählt sind: -OH, -NH₂, -COOH, -NO₂, -OR₅, -COOR₆, -NR₇R₈, wobei R₅, R₆, R₇ und R₈ die oben angegebenen Bedeutungen aufweisen;
- Aminosäureresten und cyclischen oder nicht cyclischen Kohlenhydraten.

2. Zusammensetzung nach Anspruch 1 , **dadurch gekennzeichnet, dass** das Derivat mindestens eine und vorzugsweise alle folgenden Bedingungen erfüllt:
- R bedeutet ein Wasserstoffatom;
- OR befindet sich in Bezug auf die Iminogruppe in ortho- oder para-Stellung;
- R₄ ist eine in Anspruch 1 definierte Gruppe -NQ₄Q₅.

3. Zusammensetzung nach Anspruch 1 oder 2, **dadurch gekennzeichnet, dass** das Derivat das N'-(4-Hydroxyphenyl)-N-N-dimethyl-formamidin ist.

4. Zusammensetzung nach einem der Ansprüche 1 bis 3, **dadurch gekennzeichnet, dass** das Derivat der Formel (I) in einer Menge von 0,001 bis 10 % und vorzugsweise 0,005 bis 5 % des Gesamtgewichts der Zusammensetzung enthalten ist.

5. Zusammensetzung nach einem der Ansprüche 1 bis 4, **dadurch gekennzeichnet, dass** die Zusammensetzung ferner mindestens einen Wirkstoff enthält, der unter den Keratolytika und/ oder exfoliativen Wirkstoffen, entzündungshemmenden Mitteln, reizlindernden Stoffen, anderen depigmentierenden Wirkstoffen und deren Gemischen ausgewählt ist.

6. Verwendung mindestens eines Derivats der Formel (I) nach einem der Ansprüche 1 bis 3 in einer die menschliche Haut, das Körperhaar oder die Haare depigmentierenden und/oder bleichenden kosmetischen Zusammensetzung.

7. Verwendung mindestens eines Derivats der Formel (I) nach einem der Ansprüche 1 bis 3 als Tyrosinase-Inhibitor und/oder Inhibitor der Melanin-Synthese in einer kosmetischen Zusammensetzung.

8. Verwendung mindestens eines Derivats der Formel (I) nach einem der Ansprüche 1 bis 3 zur Herstellung einer dermatologischen Zusammensetzung, die dazu vorgesehen ist, die menschliche Haut zu depigmentieren und/oder zu bleichen und/oder Pigmentflecken der Haut zu entfernen und/ oder das Körperhaar und/ oder die Haare zu depigmentieren.

9. Kosmetisches Verfahren, um die menschliche Haut, das Körperhaar oder die Haare zu depigmentieren und/oder zu bleichen, **dadurch gekennzeichnet, dass** es darin besteht, auf die Haut, das Körperhaar oder die Haare eine Zusammensetzung nach einem der Ansprüche 1 bis 5 aufzutragen.

## Claims

1. Composition suitable for topical application to the skin and/or its superficial growths comprising, in a cosmetically and/or dermatologically acceptable medium, at least one derivative of following formula (I): 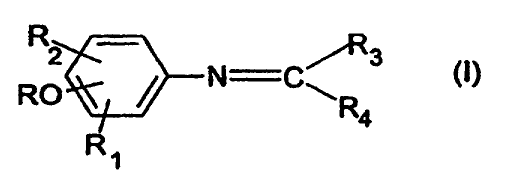 in which:
• R represents a group chosen from:
- the hydrogen atom;
- a saturated or unsaturated, linear, branched or cyclic C₁-C₂₄ alkyl group which is optionally hydroxylated by one or more hydroxyl functional groups;
- an aryl group which is unsubstituted or substituted by one or more functional groups chosen from: -OH, NH₂, -COOH, -NO₂, -OR₅ with R₅ = C₁-C₂₄ alkyl, -COOR₆ with R₆ = C₁-C₂₄ alkyl, or -NR₇R₈ with R₇ = H or C₁-C₂₄ alkyl and R₈ = H or C₁-C₂₄ alkyl;
- a -COR₉ group, R₉ representing a saturated or unsaturated, linear, branched or cyclic C₁-C₂₄ alkyl group which is optionally hydroxylated by one or more hydroxyl functional groups or an aryl group which is unsubstituted or substituted by one or more functional groups chosen from -OH, -NH₂, -COOH, -NO₂, -OR₅, -COOR₆ or -NR₇R₈ in which R₅, R₆, R₇ and R₈ have the same definition as above;
• R₁ and R₂ which are identical or different, represent a group chosen from:
- the hydrogen atom;
- a saturated or unsaturated, linear, branched or cyclic C₁-C₂₄ alkyl group which is optionally hydroxylated by one or more hydroxyl functional groups;
- an aryl group which is unsubstituted or substituted by one or more functional groups chosen from -OH, -NH₂, - COOH, -NO₂, -OR₅, -COOR₆ or -NR₇R₈ in which R₅, R₆, R₇ and R₈ have the same definition as above;
- a group chosen from: -OH, -OQ₁, -COQ₂, -COOQ₃, -NQ₄Q₅, -CONQ₆Q₇, -SQ₈ or -CH₂OQ₉, Q₁, Q₂, Q₃, Q₄, Q₅, Q₆, Q₇, Q₈ and Q₉ being chosen from the hydrogen atom, saturated or unsaturated, linear, branched or cyclic C₁-C₂₄ alkyl groups which are optionally substituted by one or more hydroxyl groups or aryls which are unsubstituted or substituted by one or more functional groups chosen from: -OH, -NH₂, -COOH, -NO₂, -OR₅, -COOR₆ or -NR₇R₈ in which R₅, R₆, R₇ and R₈ have the same definition as above;
- residues of amino acids and of carbohydrates which are cyclic or non-cyclic;
• R₃ represents a hydrogen atom;
• R₄ represents a group chosen from:
- the hydrogen atom;
- an aryl group which is unsubstituted or substituted by one or more functional groups chosen from -OH, -NH₂, -COOH, -NO₂, -OR₅, -COOR₆ or -NR₇R₈ in which R₅, R₆, R₇ and R₈ have the same definition as above;
- a group chosen from: -OH, -OQ₁, -COQ₂, -COOQ₃, -NQ₄Q₅, -CONQ₆Q₇, -SQ₈ or -CH₂OQ₉, Q₁, Q₂, Q₃, Q₄, Q₅, Q₆, Q₇, Q₈ and Q₉ being chosen from the hydrogen atom, saturated or unsaturated, linear, branched or cyclic C₁-C₂₄ alkyl groups which are optionally substituted by one or more hydroxyl groups or aryls which are unsubstituted or substituted by one or more functional groups chosen from: -OH, -NH₂, -COOH, -NO₂, -OR₅, -COOR₆ or -NR₇R₈ in which R₅, R₆, R₇ and R₈ have the same definition as above;
- residues of amino acids and of carbohydrates which are cyclic or non-cyclic;

2. Composition according to Claim 1, **characterized in that** the said derivative exhibits at least one and preferably all of the conditions below:
- R represents a hydrogen atom,
- OR is in the ortho or para position with respect to the imine functional group,
- R₄ represents an -NQ₄Q₅ group as defined in Claim 1.

3. Composition according to Claim 1 or 2, **characterized in that** the said derivative is N'-(4-hydroxyphenyl)-N,N-dimethylformamidine.

4. Composition according to any one of Claims 1 to 3, **characterized in that** the derivative of formula (I) is present in an amount ranging from 0.001 to 10% and preferably from 0.005 to 5% of the total weight of the composition.

5. Composition according to any one of Claims 1 to 4, **characterized in that** the composition additionally comprises at least one active principle chosen from keratolytic and/or desquamative agents, antiinflammatory agents, soothing agents, other depigmenting agents and their mixtures.

6. Use of at least one derivative of formula (I) as defined in one of Claims 1 to 3 in a cosmetic composition which depigments and/or lightens the human skin, the body hairs or the hair.

7. Use of at least one derivative of formula (I) as defined in one of Claims 1 to 3 in a cosmetic composition as inhibitor of tyrosinase and/or of the synthesis of melanin.

8. Use of at least one derivative of formula (I) as defined in one of Claims 1 to 3 in the manufacture of a dermatological composition intended to depigment and/or lighten the human skin and/or to remove pigmental blemishes from the skin and/or to depigment the body hairs and/or the hair.

9. Cosmetic process for depigmenting and/or lightening the human skin, the body hairs or the hair, **characterized in that** it consists in applying a composition according to one of the preceding Claims 1 to 5 to the skin, the body hairs or the hair.
